# EUROPEAN PATENT SPECIFICATION

(11) **EP 0 308 242 B1**
(45) Date of publication and mention of the grant of the patent: **04.01.1995**
(21) Application number: 88308590.4
(22) Date of filing: 16.09.1988
(51) Int. Cl.: G01N 33/555, G01N 33/563, G01N 33/531, G01N 33/569, G01N 33/80, G01N 33/94

(54) **Agglutination assay**
Agglutinationstest
Essai d'agglutination

(30) Priority: 17.09.1987 AU 4400/87; 22.10.1987 AU 5018/87; 22.10.1987 US 111313; 13.01.1988 US 143343
(43) Date of publication of application: 22.03.1989
(73) Proprietor: AGEN LIMITED, Acacia Ridge Queensland 4110 (AU)
(72) Inventor: Hillyard, Carmel J., Brisbane Queensland 4000 (AU); Bundesen, Peter Gregory, Fig Tree Pocket Queensland 4069 (AU); Rylatt, Dennis B., Rosalie Queensland 4064 (AU); Kemp, Bruce E., Kew Victoria 3101 (AU)
(74) Representative: Ritter, Stephen David

(56) References cited:
- EP-A- 0 074 271
- EP-A- 0 096 463
- EP-A- 0 143 574
- WO-A-85/04811
- WO-A-88/03650

## Description

The present invention relates to a reagent and a method for detecting an antigen, antibody or other analyte in human or animal blood by erythrocyte agglutination. The invention also concerns a kit containing the reagent and processes of preparation of the reagents.

### BACKGROUND ART

Assaying blood samples for a particular antigen or antibody has traditionally involved the step of separating the cellular components from the serum components of the blood by centrifugation and/or clotting, prior to assay.

This presents several potential problems. Firstly, such an assay is not suited to testing being conducted under field conditions. In many veterinary situations a quick test in the field is more desirable than the alternative of transporting samples to laboratories for separation and assay. Also, veterinary surgeons who do not have access to a centrifuge frequently need to assay blood samples for the presence of infectious agents such as heartworm. Further, assays being used for the detection of diseases in Third World countries present a situation where simplicity and low cost are of the essence.

Secondly, in certain pathologic conditions, separation of the blood samples becomes difficult. Blood taken from patients suffering conditions such as Waldenstrom's macroglobulinemia is difficult to separate into serum and cell fractions making an assay which can be conducted or whole blood highly desirable.

Thirdly, blood samples are often used for testing for the presence of highly contagious and potentially dangerous disease states. In these cases it is preferable that as little handling and processing of the samples as possible is undertaken in order to minimize the risk to personnel conducting the assay. Further, certain conditions make the provision of over-the-counter finger-prick assays highly desirable. Such assays must necessarily be suited to performance on whole blood.

Immunoassays have revolutionized human diagnostic and veterinary medicine since the introduction of techniques such as the radioimmunoassay, first reported by Yalow and Berson (1959) Nature 184, 1648, and the enzyme immunoassay or EIA which was first reported by Engvall and Perlman (1971) Immunochem 8, 871 and Van Neeman and Schuurs (1971) FEBS Letters 15, 232.

Whilst such assays are based on antibody-antigen interactions the detection systems utilized are usually complex. The reagents used are generally enzyme or radiolabelled antigens, antibodies or complexes thereof which require either incubation With specific substrates and measurement of a color end-point either visually or by means of a colorimeter or measurement of radioactive decay with radiation counters to detect the presence of the analyte being tested. These assays also involve several washing steps. Most immunoassays for the detection of analytes in blood are currently of this nature. Thus, whilst these assays are sensitive, they are lengthy and involve procedures which may require expensive instrumentation, for detection of the analyte under test.

An alternative to these assays is provided by immunoassays of the type described by Gupta, et al., (1985) Journal of Immunological Methods 30 177-187. These are immunoassays in which erythrocytes and anti-erythrocyte antibodies are used in the indicator system. In these assays exogenous erythrocytes such as sheep erythrocytes are used.

In recent years it has been possible to attach antibodies to latex beads, thus providing a rapid agglutination assay. This, however, still entails the separation of the serum/plasma phase from the cellular phase and consequently requires the use of a centrifuge or filtration system. Latex agglutination assays are described in Merskey, et al., Proc. Soc. Exp. Biol . Med. (1969), 131, 871; Castelon, et al., J. Clin. Pathol. (1968), 21, 638; and Singer & Plotz AM. J. Med. [1956 (Dec)], 888.

Both direct and indirect agglutination immunoassays are well known in the art. In these assays, the agglutination of particles to which antigen or antibody is bound is used to indicate the presence or absence of the corresponding antibody or antigen. A variety of particles, including particles of latex, charcoal, kaolinite, or bentonite, as well as both microbial and red blood cells, have been used as agglutinatable carriers. See Mochida, US 4,308,026. The use of erythrocytes as indicator particles is strongly criticised by Patel, US 3,882,225, who says that it is difficult to standardize indicator erythrocytes.

Molinaro, US 4,130,634 describes an assay for an antigen which employs antibody-coated red blood cells. Molinaro emphasizes that the method used to couple the antibody to the erythrocyte must not destroy the reactivity of the antibody. He makes it clear that antibodies which are specific for the erythrocyte are not useful for his assay. He does mention, however, the possibility of using a hybrid antibody with one binding site specific for the antigen and the other specific for the red blood cell.

Chang, US 4,433,059 discloses an agglutination immunoassay reagent in which two antibodies are covalently linked "tail-to-tail", i.e., so as not to alter their specificity. One antibody is specific for an antigen borne by an indicator substance, such as an erythrocyte. This antibody is preferably univalent to avoid nonspecific agglutination. The other antibody is divalent and is specific for the analyte. In preparation for the assay, fresh erythrocytes are coated with the conjugate. The double antibody conjugate-coated RBCs are then incubated with the test serum. Chang does not contemplate the assaying of whole blood samples using a non-autoagglutinating anti-RBC antibody and endogenous erythrocytes.

Chu, US 4,493,793 discloses the construction of a lectin-antibody or lectin-antigen covalently coupled conjugate. His Table I sets forth the carbohydrate specificities of several lectins. He does not teach coupling such a conjugate to an erythrocyte through either the lectin or the antibody receptor.

Other "tail-to-tail" immunological conjugates are known. Segal , US 4,676,980 sets forth the construction of a "tail-to-tail" conjugate of a target cell surface antigen-specific antibody and of a cytotoxic effector cell receptor-specific antibody. Several cross-linking methods, are described. This conjugate is intended for use in immunotherapy, in that it will cause the cellular immune system of the patient to lyse the target cell. The target cell would not, of course, be an erythrocyte endogenous to the host.

Li , US 4,661,444 suggests the production of a tail-to-tail conjugate of an analyte-binding antibody and of an antibody specific for the idiotype of the first antibody. This conjugate was to be used in conjunction with an insolubilized analyte-binding antibody in an immunoassay.

Wardlaw, US 4,695,553 teaches use of a monoclonal antibody against a universal erythrocyte antigen as a RBC agglutinating agent to clarify the interface between red blood cells and white blood cells in centrifuged whole blood. He prefers use of antibodies against glycophorin or against H antigen, but also mentions the possibility of using a mixture of lectins. Guesdon, US 4,668,637 discusses the use of anti-red blood cell antibodies or of lectins for the purpose of erythroadsorption. Bigbee, [Molecular Immunology, 20: 1353-1362 (1983)] describes the production and testing of four monoclonal antibodies against glycophorin A. The general concept of using in an immunoassay an antibody which reacts with an antigenic determinant shared among all members of a class of analytes of interest (microorganisms) is set forth in McLaughlin, US 4,683,196.

A number of patents deal with antibodies useful in blood typing. See, e.g., Lloyd, US 4,678,747; Graham, Jr., US 4,358,436; Liu, US 4,550,017; Steplewski , US 4,607,009; Lennox, WO83/03477. These antibodies are useful for blood typing because they bind to antigens found only in certain blood cell populations, while for the purpose of this invention, it is desirable to use antibodies (or mixtures thereof) which bind to essentially all erythrocytes.

Zuk, US 4,594,327 recognizes the desirability of performing an immunoassay directly on whole blood samples. In his method, the sample is contacted with both an insolubilized, analyte-specific immunoreagent and with a red blood cell binding agent such as a RBC-specific antibody or a lectin. The analyte-specific immunoreagent and the RBC binding agent are not coupled together, and the assay disclosed is not an agglutination assay.

The problem, in an agglutination immunoassay, of nonspecific agglutination of erythrocytes by anti-erythrocyte antibodies endogenous to the blood sample, was noted by Czismas, US 3,639,558. He proposed eliminating all naturally occurring antigenic sites on the particle by coating the particle with protein.

Theofilopoulos, US 4,342,566; Duermeyer, US 4,292,403 and Goldenberg, US 4,331,647 are of interest as demonstrating the use of specific binding fragments of antibodies as substitutes for intact antibodies in assays. The construction of heterobifunctional antibodies is taught by Auditore-Hargreaves, US 4,446,233; Paulus, US 4,444,878; and Reading, US 4,474,893. Mochida, US 4,200,436 discloses the use of monovalent antibodies or binding fragments thereof in certain immunoassays. Forrest, US 4,659,878 mentions that monovalent antibodies cannot form dimers or more extensive complexes with the antigen; such aggregates were said to be capable of interfering with the binding of the antigen-antibody complex to a solid phase support.

### DESCRIPTION OF THE INVENTION

The present inventors recognized that there was a need for a method which can be used in the laboratory and in the field, particularly in Third World Countries where there is lack of medical testing facilities for analysis of different types of analytes in whole blood. As indicated above, earlier methods require separation of the blood cells from serum or plasma and are therefore difficult and in many cases impossible to implement in the field.

The present invention is based on the finding that if erythrocyte-binding molecules are coupled to specific analyte-binding molecules, then the resulting conjugate could be used to bind both endogenous erythrocytes, and analytes present in a blood sample. The present invention results from the finding that when such a complex is exposed to a blood sample, agglutination of the erythrocytes endogenous to that sample will serve as an indicator of the presence of the relevant analyte (usually, an antigen or antibody) due to cross-linking of erythrocytes with the analyte.

Thus according to one aspect of the invention there are provided agglutination reagents for direct detection of an analyte in a blood sample, comprising an erythrocyte binding molecule coupled to an analyte binding molecule, wherein the erythrocyte binding molecule is coupled to the analyte binding molecule in such a manner that the binding characteristics of the binding molecules are not altered by the coupling, and the erythrocyte binding molecule is capable of binding erythrocytes endogenous to the blood sample but does not agglutinate endogenous erythrocytes in the absence of analyte.

Such agglutination reagents may be used in a direct agglutination assay for the presence of an analyte in a blood sample containing erythrocytes which assay comprises mixing the sample with an agglutination reagent as defined above for a time sufficient for binding to occur to analyte and erythrocytes to cause agglutination; observing whether the erythrocytes are agglutinated and correlating the agglutination with the amount of analyte present in the sample.

The invention further provides agglutination reagents for indirect detection of an analyte in a blood sample, comprising an erythrocyte binding molecule coupled to an analyte analogue wherein said erythrocyte binding molecule is coupled to said analyte analogue in such a manner that binding characteristics of the erythrocyte binding molecule are not altered by the coupling, and said erythrocyte binding molecule is capable of binding erythrocytes endogenous to the sample but does not agglutinate endogenous erythrocytes in the absence of an analyte binding reagent.

These agglutination reagents may be used in an indirect agglutination assay for the presence or amount of an analyte in a blood sample which comprises incubating the sample with an agglutination reagent as defined above and a soluble analyte binding reagent, whereby said agglutination reagent competes with sample analyte for the analyte binding sites of said analyte binding reagent, wherein the incubation is for a time sufficient for analyte to prevent binding of said analyte binding reagent to said analyte analogue to prevent agglutination, observing whether agglutination occurs, and determining the presence or amount of said analyte from the inverse of the degree of agglutination.

Test kits comprising the aforementioned agglutination reagents together with a reference solution containing a known quantity of analyte are further provided according to the invention.

Advantages of the reagents, assays and test kits are summarized as follows:

### Advantages of endogenous RBCs

### i) Simplifies current assay procedures

- no need to centrifuge sample; whole blood, collected in the presence of a suitable anticoagulant, is used instead of serum or plasma.
- for samples from patients with infectious diseases, such as AIDS or hepatitis, there is minimal sample handling.
- appropriate for mass screening programs as conducted by the World Health Organization in third world countries, whose facilities are limited.
- the assay is very robust; there is only a single reagent, which is stable in the presence of a bacteriostatic agent such as 0.01% (w/v) sodium azide.
- can be used as a field test by veterinary practitioners, when the appropriate animal red cells are used for immunization to produce species specific MAb.
- the test is very fast - agglutination occurs in less than three minutes.
- the method can be used to monitor therapeutic drugs and patient compliance.
- it also has possible use as an "over-the-counter" self testing assay.
- the only equipment needed is a mixing stick, glass or plastic slide, lancet and possibly a microcapillary.

### ii) Advantages over exogenous erythrocytes include:

- no pretreatment of erythrocytes. Patent 4,433,059 uses blood group O negative cells, which have been spun down, reacted with antibody conjugate for 15-30 minutes and washed 3x in PBS. Patent 4,668,647 uses sheep red blood cells, which have been washed and resuspended in PBS. After the reaction, which takes place on a solid support, the cells are then fixed.
- no pretreatment of samples. Patent 4,433,059 notes that samples have to be heat inactivated to avoid interference due to complement. Rabbit serum and bovine albumin must also be added to minimize other non-specific reactions. None of this is necessary with the present system, where undiluted whole blood from patients may be reacted directly with reagent. This reagent contains unrelated monoclonal antibody to prevent any anti-mouse reactions, which may occur.

Thus, it is possible to dispense with the cumbersome separation of cells from serum and with the sensitization and fixing of exogenous erythrocytes intended for use as indicator particles in agglutination assays. The endogenous erythrocytes are sensitized by the reagent.

Another novel aspect of applicants' agglutination reagents and assays is the selection of an erythrocyte binding molecule such treat incubation of conjugate with endogenous erythrocytes will not cause agglutination of the erythrocytes unless analyte is also present -- such erythrocyte binding molecules are termed herein "non-auto-agglutinating".

The erythrocyte binding molecule is preferably a monoclonal antibody and especially, in the human system, an anti-glycophorin antibody. It is believed that this antibody is non-autoagglutinating for steric reasons; either the binding sites of the intact antibody are able only to bind adjacent epitopes on the same erythrocyte or only one of the two binding sites can bind to glycophorin at one time.

Applicant's assay can detect small antigens without repeating determinants, using two conjugates, one bearing an analyte-specific binding molecule and the other, a binding molecule specific for a new epitope formed by the binding of the first conjugate to the analyte. This allows cross-linking in the presence of the antigen to be measured.

### Brief Description of the Drawings

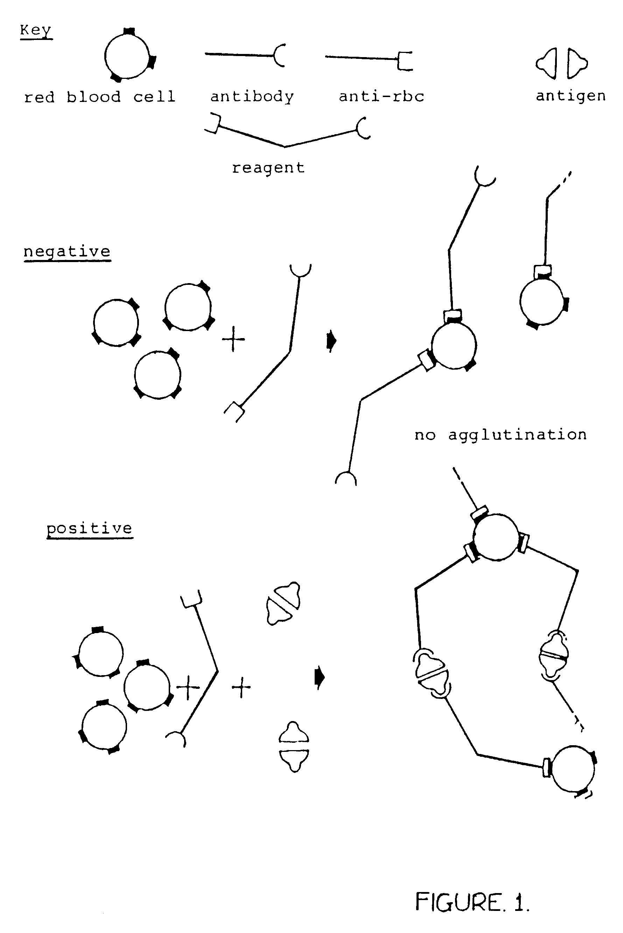

Fig. 1 is a schematic representation of erythrocytes showing positive and negative agglutination results with antibody complexes in the presence and absence of antigen respectively.

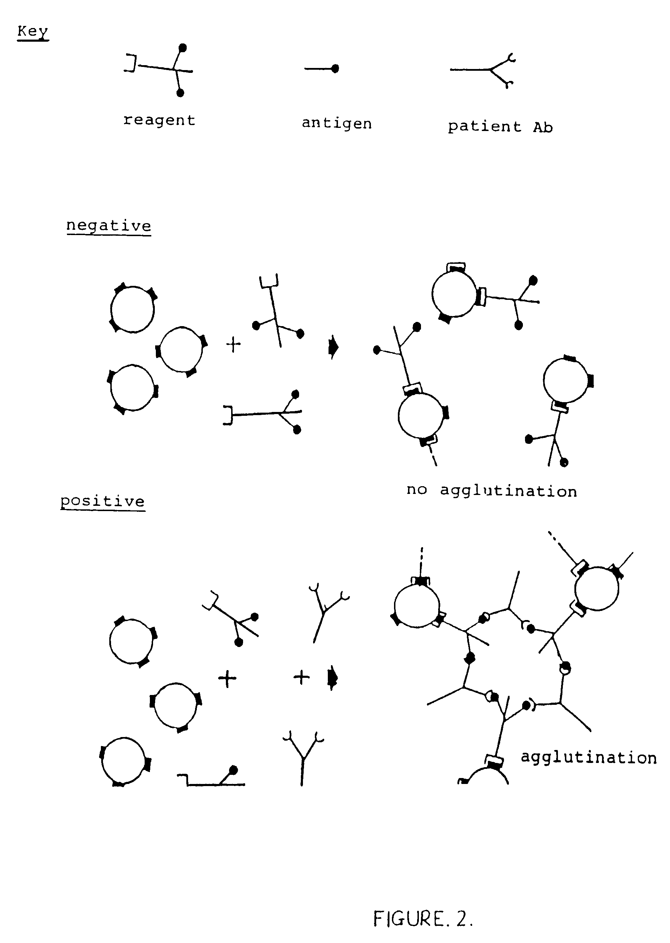

Fig 2. is a schematic representation of erythrocytes showing positive and negative agglutination results with a complex of antibody and an antigen in the presence and absence, respectively of anti-antigen antibodies.

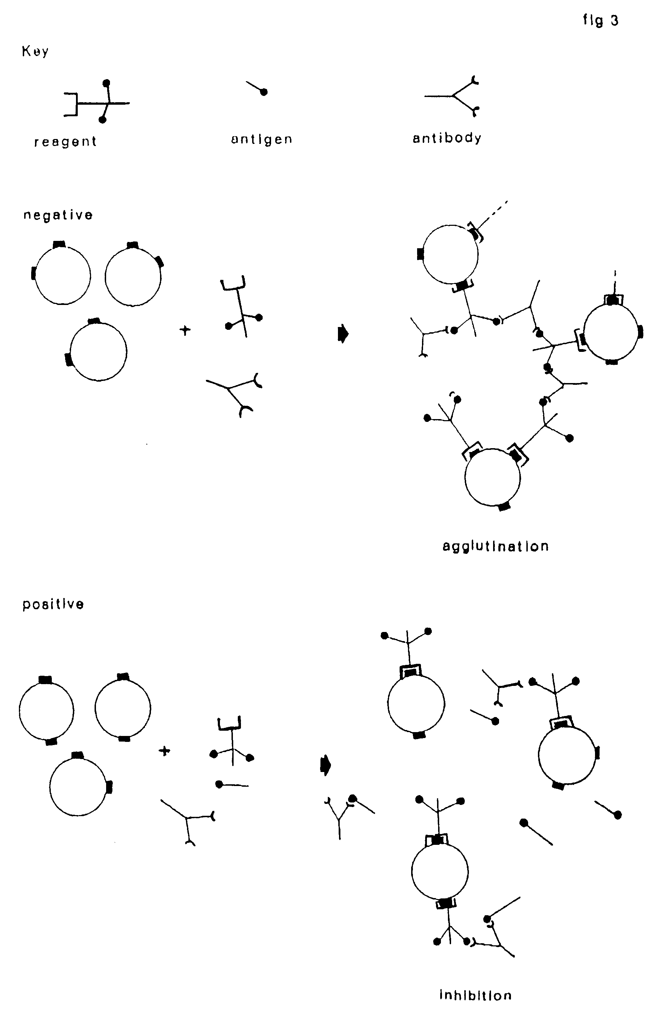

Fig 3. is a schematic representation depicting (a) erythrocyte agglutination and (b) inhibition of erythrocyte agglutination due to presence of analyte or antigen.

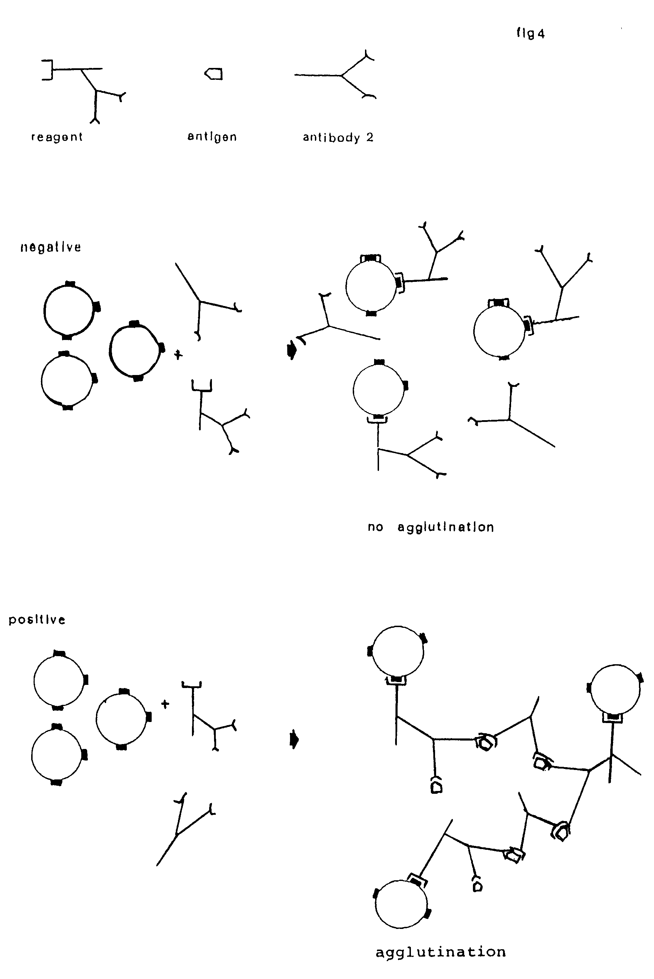

Fig 4. is a schematic representation depicting mechanisms of agglutination/non-agglutination in connection with an overlapping antigen assay.

### DETAILED DESCRIPTION OF THE INVENTION

In the agglutination assay of this invention, a reagent is provided which comprises an erythrocyte binding portion provided by an erythrocyte binding molecule attached to an analyte binding portion provided by an analyte binding molecule, or to an analyte analogue, without substantially changing the binding characteristics of the binding portions. The reagent is non-agglutinating when incubated with endogenous erythrocytes in the absence of the analyte.

### Erythrocyte Binding Molecules

Erythrocyte membranes contain various antigenic surface constituents, including proteins, glycoproteins, glycolipids and lipoproteins. Antibodies which recognize these constituents may be prepared by conventional techniques using the membrane, or the purified constituents thereof, as immunogens. These antibodies may be monoclonal or polyclonal in nature. Either the intact antibody, or specific binding fragments thereof, may be used as erythrocyte binding molecules (EBM). The antibody or antibody fragment may be polyvalent, divalent or univalent.

In addition, glycoproteins, glycolipids and other carbohydrate structures on the surface of erythrocytes are recognized by chemicals known as lectins, which have an affinity for carbohydrates. These lectins may also be used as EBMs. Other receptor molecules with specific affinity for the erythrocyte surface also may be used. These could also include molecules with an affinity for the lipid bilayer of the membrane. Examples of such molecules are: protamine, the membrane binding portion of the bee venom, melittin, and other very basic peptides.

The preferred EBMs of the present invention will recognize erythrocyte membrane constituents found on all, or nearly all erythrocytes, so that erythrocytes endogenous to the blood sample may be used as the agglutinating particles. Such constituents include the so-called "public antigens".

Erythrocyte membranes are lipid bilayers with a variety of proteins either on the surface or with a hydrophobic portion allowing the protein to anchor in or pass through the membrane, and may have part of the molecule inside the cell.

Glycophorin A is an example of a molecule which traverses the cell membrane. The blood group specificity is conferred by carbohydrate or glycolipid moieties, which are attached to membrane proteins. It is thus important that an EBM should recognize either the protein part of a membrane glycoprotein constituent, which is common to all erythrocytes of a particular species or another common structure. The ability of a bivalent EBM to agglutinate red cells will depend on steric factors, such as the mobility of the molecule and the position of the binding site above the lipid bilayer.

The proteins of erythrocyte membranes include:- glycophorin A (MN, En^{a}, Wr^{b}), glycophorin B (Ss, 'N', U) and the minor constituents, integral membrane protein 1 (Rhesus), membrane attached glycoprotein C4 (Chido & Rodgers), integral membrane glycoprotein (anion channel), glycolipids (Lewis), glycosphingolipids (ABH, li, P, Tk), ankyrin, spectrin, protein 4-1, F-actin. [The associated blood group factors are in parentheses.]
The following publications are also relevant:
1. The red cell membrane. S.B. Shohet & E. Beutler, in: Hematology, 3rd ed. Eds: Williams, Beutler, Erslev & Lichtman, 1983. (Review of all erythrocyte membrane antigens)
2. The red cell membrane skeleton. T. Marchesi . Blood, 61, 1-11, 1983. (Review of the skeleton proteins)
An especially preferred EBM is one recognizing glycophorin. When erythrocyte sialoglycopeptides are extracted from membranes, the main fraction (approximately 75% of total) is glycophorin. This molecule comprises 131 amino acids with 16 oligosaccharide chains. Thus, this is an abundant moiety, which could allow antibody attachment without agglutinating the red cells. It is also readily available in a relatively pure form commercially, e.g., from Sigma Chemical Company. (See "Fractionation of the Major Sialoglycopeptides of the Human Red Blood Cell Membrane" H. Furthmayr, M. Tomita & V.T. Marchesi . BBRC 65, 1975, 113-122).

When the erythrocyte binding molecule is multivalent, as in the case of a normal antibody, it is desirable that the molecule recognize an erythrocyte membrane constituent which is abundant and well-distributed, and the binding site should be in such a position that crosslinking between cells is inhibited by steric hindrance, thereby avoiding premature red cell agglutination.

It is preferable, but not necessary, that a single EBM be used that recognizes essentially all erythrocytes. Several EBMs may be used, either in the same or in separate conjugates, each of which recognizes a particular group of erythrocytes, but which in aggregate recognize essentially all erythrocytes.

While it is preferable that the EBM recognize a natural surface constituent of the erythrocyte, it is possible to coat erythrocytes with a ligand recognized by the EBM, or to treat the erythrocytes so as to expose a normally cryptic ligand.

### Analytes

This invention is not limited to the detection of any particular analyte. The analyte may be a substance normally found in blood, such as a blood protein or a hormone, or it may be a foreign substance, such as a drug (including both therapeutic drugs and drugs of abuse), or an organism, such as a virus (by recognizing a virus coat protein) bacterium, protozoan, fungus, or multicellular parasite (e.g., heartworm).

The analyte may have repeating epitopes, recognizable by one analyte binding molecule, or unique epitopes, where a mixture of analyte binding molecules is necessary. However, analytes which can only be bound by one ABM at a time, may also be detected.

### Analyte Binding Molecule

The analyte binding molecule may be any substance having a preferential affinity for the analyte, including monoclonal or polyclonal antibodies lectins, enzymes, or other binding proteins or substances (or binding fragments thereof). Where the analyte is an antigen, the ABM is usually an antibody. Where the analyte is an antibody, the ABM is usually an antigen recognized by that antibody. When the analyte to be detected has no repeating epitopes, two or more ABMs are required with different specificities for the analyte. The reagent in this case will be either a mixture of EBM bound to ABM1 and EBM bound to ABM2, or EBM with both ABMs attached.

The analyte binding molecule need not bind the analyte directly. For example, in an assay for thyroxine, one ABM may be directed against a thyroxine binding protein if the latter is known to be present in the sample, or is separately provided.

### Coupling of EBM and ABM

The EBM and the ABM may be coupled together directly or indirectly, and by covalent or non-covalent means (or a combination thereof). Where multiple EBMs or ABMs are used, EBMs or ABMs may be coupled together, with one or more ABMs coupled directly to an EBM. The following table summarizes some of the covalent coupling methods known in the art.

### Heterobifunctional

1. SPDP
   (N-Succinimidyl-3,2-(pyridyldithio)propionate)
   Neurath, et al., 1981, J. Virol., Meth., 3, 155-165.
2. MBS
   (m-maleimidobenzoyl-N-hydroxysuccinimide ester)
   Kitagaw, et al., 1976, J. Biochem., 79, 223-236.
3. SIAB
   (N-succinimidyl-4-iodoacetylaminobenzoate)
   Weltman, et al., 1983., Bio. Techniques, 1, 148-152.

### Selective Bifunctional

P-isothiocyanatobenzoylchloride
US Patent 4 680 338

### Bifunctional

1. BSOCOES
   Bis[2-(succinimidooxycarbonyloxy)ethyl]sulphone
   Zarling, et al., 1980, J. Immunol., 124, 913-920
2. BS
   Bis(sulphosuccinimidyl)suberate
   Staros, 1982, Biochemistry 21, 3950-3955.

### Other

1. Glutaraldehyde
   Avrameas, 1969, Immunochem., 6, 43.
2. Periodate Oxidation
   Nakane and Kawoi, 1974, J. Histochem. Cytochem., 22, 1084-1091.
3. Carbodiimide

Of the foregoing methods of covalent coupling, conjugation with SPDP is preferred.

The EBM and the ABM may also be coupled noncovalently, for example, by (a) attaching biotin to one and avidin (or strepavidin) to the other), (b) attaching an anti-antibody to one, which then binds the other, (c) attaching Protein A to one, which then binds the F_{c} portion of the other, and (d) attaching a sugar to one and a corresponding lectin to the other.

It should be understood that in coupling the EBM and the ABM, the binding characteristics should be changed as little as possible. It may be advantageous to provide a spacer moiety between the EBM and the ABM to reduce steric hindrance.

The EBM/ABM conjugate may be a chimeric antibody. One method of constructing such a conjugate is the following:
(a) preparing F(ab)₂ fragments of a selected antibody by pepsin digestion;
(b) reducing and treating the fragments with Ellman's reagent to produce Fab' fragments of the selected antibody;
(c) thiolysing a selected specific antibody or a selected erythrocyte antibody; and
(d) coupling the thioylated Fab' fragment to the Ellman's reagent treated Fab' fragment to produce a chimeric anti-erythrocyte antibody-antigen specific antibody conjugate.

Another method is set forth below:
(a) treating an anti-erythrocyte monoclonal antibody-producing hybridoma and an antigen specific monoclonal antibody-producing hybridoma with a distinct site-specific irreversible inhibitor of macromolecular biosynthesis;
(b) fusing the two different monoclonal antibody-producing hybridomas with polyethylene glycol
(c) cloning the fused cells either in soft agarose or by limiting dilution;
(d) selecting cloned heterohybridomas secreting chimeric anti-erythrocyte antibody-antigen specific antibody with a screening assay appropriate to the antibodies.

Preferably the inhibitor is selected from the group consisting of emetine, actinomycin D, hydroxyurea, ouabain, cycloheximide, edine and sparsomycin.

The chimeric antibody may be two half-molecules, one with specificity for erythrocytes (the EBM) and the other with specificity for the analyte (the ABM). In this case the disulfide bonds of the antibody couple the ABM to the EBM to form the conjugate. Alternatively, the two half-molecules may be specific for the same or different epitopes of the analyte. In this second case, the chimeric antibody is really two ABMs and must be coupled to an EBM to form a tripartite conjugate. Tripartite conjugates may be formed by other means, such as attaching the EBM and two ABMs to a macromolecular spacer.

The simplest agglutination reagent contemplated is one comprising a single conjugate of one EBM to one ABM. This reagent is suitable for the detection of antigens with repeating epitopes.

Antigenic analytes large enough to allow simultaneous binding of two antibody molecules, but which lack repeating epitopes, are known. They include many peptide and protein hormones. For agglutination to occur, the antigen must interact with the reagent so that at least some molecules of antigen act as a bridge between proximate erythrocytes. For assaying such analytes, it is preferably to employ a reagent comprising two or more distinct conjugates, i.e., ABM1/EBM + ABM2/EBM where ABM1 and ABM2 bind to different, non-overlapping epitopes of the analyte. One might instead use a more complex single conjugate, ABM1/ABM2/EBM, where the stereochemistry is unlikely to favor the binding of both ABMs on the same conjugate molecule to the same analyte molecule.

### Erythrocyte Agglutination Assay

Both direct and indirect agglutination assays are known in the art. In the conventional direct assay for an antigen, red cells are coated with antibody, and reacted with the sample. Multifunctional antigens act as bridges between the coated red blood cells, creating an agglutinate. In the conventional indirect assay, red cells are coated with antigen, and contacted with both a soluble antibody and with sample. Sample antigen competitively inhibits the binding of the sensitized red cells by the antibody, and hence the agglutination. It is also possible to additionally use an antibody - sensitized RBC. See Mochida, U.S. 4,308,026.

The reagent of the present invention may be used in either a direct or an indirect agglutination assay format. However, unlike conventional assays, it is not necessary to precoat erythrocytes with antibody or antigen. Rather, the reagent may be added to a blood sample containing endogenous erythrocytes, whereupon it will sensitize the cells, rendering them able to bind sample analyte (a direct assay) or to compete with sample analyte for a soluble analtye-binding molecule analyte-binding molecule (an indirect assay).

For some small circulating molecules such an synthetic or natural steroids, i.e., digoxin, theophylline, etc., or drugs of abuse, i.e., phenobarbital, cannabinoids, opioids, etc., the analyte in question may be too small to provide the two necessary antigenic epitopes for antibody binding (or other "epitopes" for recognition by other binding molecules) to allow cross-linking and subsequent erythrocyte agglutination.

For the assay of small molecules, as in drug monitoring or indeed for any other antigens, an agglutination inhibition assay is preferred. In this case, a two stage test is expected. The first stage would be addition of a reagent consisting of the analyte or analyte analogue coupled to the non-agglutinating EBM, and the second stage would be addition of an unconjugated ABM. (The two stages may be reversed). If analyte is present in the blood sample, the specific binding of the ABM to the EBM-analyte analogue conjugate will be inhibited, leading to a loss of agglutination. Otherwise, agglutination occurs.

The term "analyte analogue" includes both the analyte, and any substance also specifically bound by the ABM when such binding is competively inhibited by the analyte. The analyte analogue may be anti-idiotypic antibody raised against the antigen-binding site of an analyte-binding antibody.

For the detection of such small molecules by direct agglutination assay, at least two specific monoclonal antibodies could be used. One monoclonal antibody which is capable of binding directly to the small circulating antigen would be coupled to the erythrocyte binding molecule. The second (secondary) monoclonal antibody would be incubated with the above conjugate and the analyte and would be capable of binding to a new antigenic determinant comprised of an overlapping region of the first monoclonal antibody and the antigen that exists only when the first monoclonal antibody binds antigen. Thus, the second monoclonal antibody acts as the erythrocyte "bridge", finally causes cross-linking between different red cells allowing agglutination to occur. This method, of course, is not restricted to monoepitopic analytes.

For stereochemical reasons, it may be difficult for a single secondary antibody molecule to bind simultaneously to two conjugate: analyte complexes. Thus, it may be preferable to conjugate the secondary antibody with an erythrocyte binding molecule.

In stating that a sample is to be incubated with a plurality of reagents it is to be understood that the contact may be simultaneous or sequential, without limitation to any particular order or duration of contact.

### EXAMPLE 1 - Preparation of erthyrocyte binding molecule (anti-glycophorin antibody)

### Immunization and Screening Procedure

Mice were immunized with human red blood cells and monoclonal antibodies produced by fusing the speen cells of immunized animals with mouse myeloma cells. The antibodies were screened by both spin agglutination assay and enzyme immunoassay, where glycophorin was bound to a microtitre plate. Spin agglutination was performed by a modification of Wyatt & Street, Aust. J. Med. Lab. Sci, 4 48-50. 50 µl of cell culture supernatant was mixed with 50 µl of a 1% red blood cell suspension in a microtitre plate. For this example, antibodies which bound glycophorin, but did not agglutinate, were selected. The reaction of monoclonal antibody and glycophorin was determined by enzyme immunoassay. Microplates were coated with 10 micrograms/ml human glycophorin [Sigma Cat. No. G 7389] and washed, then incubated with serial dilutions of monoclonal antibody. After further washing, the presence of bound antibody was determined by the addition of enzyme labelled anti-mouse antibodies followed by the addition of substrate. The titre was determined to the largest dilution of monoclonal , which gave an A420 reading greater than 0.1 OD units above background.

Of 384 wells, 40 primary clones were chosen. These gave either a positive spin agglutination assay, a response to glycophorin on EIA or both.

| EIA | Spin agglutination | Number of clones |
|---|---|---|
| Negative | Positive | 4 |
| Positive | Positive | 20 |
| Positive | Negative | 16 |

Subsequent absorption studies were performed to confirm that the antibodies recognized a glycophorin domain exposed on the red cell surface.

The results of the screening assays on ascitic fluid are listed below:

| Ascitic Fluid Titre | | | |
|---|---|---|---|
| Clone | Spin Agglutination | Glycophorin EIA | Red Cell Absorption Test |
| RAT 1D3/167 | 512000 | <1000 | Positive |
| RAT 3D6/5 | 6400 | 1024000 | Positive |
| RAT 1C3/86 | <1000 | 1024000 | Positive |
| RAT 3B1/172 | 256000 | 2000 | Positive |
| RAT 3D3/22 | 4000 | 1024000 | Positive |
| RAT 3D5/61 | 128000 | 1024000 | Positive |
| RAT 1A2/187 | <1000 | 256000 | Positive |
| RAT 2A2/187 | <1000 | 128000 | Positive |
| RAT 1A3/129 | <1000 | 12800 | Weak |
| RAT 1C4/5 | <1000 | 128000 | Positive |
| RAT 4C3/13 | <1000 | 128000 | Positive |
| RAT 3B1/70 | <1000 | 517000 | Positive |

RAT IC3/86 has been deposited under the Budapest Treaty, with the designation G 26.4. IC3/86, ATCC HB9893 at the American Type Culture Collection, 12301 Parklawn Drive, Rockville MD, 20852, USA on 7 September 1988.

### Purification of RAT 1C3/86

Monoclonal antibodies were purified to homogeneity from ascitic fluids by chromatography on hydroxylapatite (Stanker, et al., J. Immunol. Methods 76, 157, 1985).

### EXAMPLE 2: Preparation of HIV peptide - Ab conjugate

The spread of the human immuno deficiency virus (HIV-1) has become a major global health problem. At present there is no recognised cure or vaccine for this disease. The diagnosis of infected individuals is a major factor in attempts to curtail the spread of the virus. Moreover, the need to prevent blood product contamination and protect health care personnel has increased the demand for simple, rapid, inexpensive and specific tests for the presence of anti-HIV antibodies.

We have made use of the patient's own red cells to provide a potential detection system for anti-HIV antibodies. This has been accomplished by selecting a non-agglutinating monoclonal antibody to human red blood cells. Chemically cross-linking this antibody with a synthetic HIV peptide antigen permitted specific agglutination of patients' red cells in the presence of antibodies to this antigen. The synthetic peptide antigen derived from gp41 of HIV-1 (residues 579-602), was chosen on the basis of the Welling procedure, FEBS LETT. 188: 215 (1985) and corresponds with the region identified as a major epitope recognised by antibodies from approximately 98% of AIDS patients. [Wang et al Proc. Nat. Acad. Sci. USA 83: 6259 (1986)].

Synthetic peptides were synthesized using the Merrifield procedure [RS Hodges and RB Merrifield, Amal. Biochem. 65, 241 (1975)] with the aid of an Applied Biosystems Model 430 synthesizer using double coupling cycles supplied by the manufacture. The N-t-butyloxycarbonyl amino acid derivatives were obtained from the Protein Research Foundation (Osaka, Japan). Side chain protection was the same as supplied by Applied Biosystems with the exception of arginine for which the omega-NO₂ derivative was used. Chain assembly was monitored using ninhydrin [V Sarin et al Anal. Biochem. 117, 147 (1981)]. The assembled peptides were simultaneously cleaved and deprotected using anhydrous HF containing 10% anisole (v/v) [JM Stewart and JD Young, Solid Phase Peptide Synthesis, pp44 and 66, WH Freeman, San Francisco (1966)]. The crude peptide was precipitated with diethylether, washed with ethylacetate and extracted with 60% acetonitrile in 0.1% trifluoroacetic acid (v/v). Synthetic peptides were purified by preparative reverse phase chromotography (Amicon C₁₈ resin, 250A pore size 25 x 400 mm) , eluting with a gradient of 1,000ml, 0 to 60% acetonitrile in 0.1% trifluoroacetic acid. The synthetic peptide was approximately 95% pure as judged by analytical reversed phase HPLC and by quantitative amino acid analysis following acid hydrolysis.

### 1. SPDP labelling of the erythrocyte binding Ab (RAT 1C3/86)

To 0.25 ml of 13.8 mg/ml RAT 1C3/86 was added 12.5 µl of 2 mg/ml SPDP in dimethyl formamide and the reaction was allowed to proceed for 1 hour at 25°C. Unreacted SPDP was removed by gel filtration on Sephadex G 25 and the level of SPDP labelling (1.4 moles/mole) was determined.

### 2. Reduction of peptide 3.2

Peptide 3.2 (sequence RILAVERYLKDQQLLGIWGCSGK, corresponding to residues 579-601 of the major coat protein of HIV 1) was dissolved in 1 ml of 100mM Tris HCL, 1mM EDTA pH 8.0 and reacted with 10 µl of 2-mercaptoethanol for 45 minutes at 40°C. The reaction was terminated by the addition of 4 drops of trifluoroacetic acid (TFA) and 1 ml of aqueous 0.1% TFA. The mixture was applied to a Sep-pak (Waters) C 18 cartridge that had been treated with 20 ml of 60% acetonitrile. 0.1% TFA and equilibrated with 0.1% TFA. The reduced peptide was cycled through the Sep-pak® twice before washing with 20 ml 0.1% TFA. The reduced peptide was eluted from the Sep-pak® with 2 x 2 ml of 60% acetonitrile, 0.1% TFA. The sample was rotary evaporated to dryness prior to coupling.

### 3. Conjugation

The peptide was dissolved in 0.2 ml of a buffer containing 100mM potassium phosphate, 100mM sodium chloride and 4M guanidine HCl pH 7.4 and mixed with 2.2 mg of SPDP of labelled antibody in the same buffer, but without guanidine HCl . The flask was incubated overnight at 25°C.

The degree of substitution of the antibody influenced the solubility of the conjugate; 20 moles peptide per mole of conjugate became insoluble. The range 5-7 moles of peptide per mole antibody was optimal . The capacity of the conjugate to bind red blood cells was monitored using the agglutination test with rabbit antimouse antibody and with HIV positive whole blood.

### 4. Gel Filtration Chromatography

Unreacted peptide and SPDP by-products were removed ay gel filtration on a Superose® 6 column (Pharmacia) in phospate buffered saline and antibody containing fractions were pooled and stored at 4°C after addition of 0.01% sodium azide as a preservative.

### 5. Preparation of reagent for assay

Two volumes of conjugates were mixed with one volume of a 10 mg/ml solution of an unrelated monoclonal antibody (Bruce 5) prepared as described in Bundesen, et al., Vet. Immun., Immunopath. 8, 245-260, 1985.

### Assay procedure

For assay, 10 µl of heparinized whole blood was placed on a glass slide. 30 µl of reagent was added and mixed. The slide was rocked for up to three minutes and the presence or absence of agglutination noted.

Nine independent peptide/antibody conjugates were prepared and found to be active in agglutinating seropositive patient's red blood cells. Active conjugate was also prepared using m-Maleimidoberzoyl-N-hydroxysuccinimide ester as the cross-linking reagent.

Results were at least comparable in accuracy to those observed with an enzyme immuno assay using a similar antigen (Table 1).

Comparative testing of blood samples was by means of ELISA for the purpose of confirming positives and negatives obtained with the erythrocyte assay.

Control blood samples comprised ELISA negative blood samples and ELISA positive samples from infected patients. HIV positive patients were confirmed western blot positive by the Victorian State Reference Laboratory. Fairfield hospital patients were negative either by western blot or EIA (Abbot Laboratories). Blood donors were tested by EIA (Genetic Systems). False positive or negative values are given in parentheses and were verified by EIA or western blot analysis.

**TABLE 1**

| Autologous Red Cell Agglutination Test | | | | |
|---|---|---|---|---|
| | Agglutination Test | | EIA Test | |
| | +ve | -ve | +ve | -ve |
| HIV +ve patients | 42 | (1) | 43 | 0 |
| Fairfield Hospital patients | (3) | 63 | 0 | 66 |
| Healthy blood donors | (1) | 873 | (2) | 872 |

In order to evaluate the specificity of the test a series of synthetic peptides corresponding to other regions of the HIV-1 envelope proteins was tested for their capacity to inhibit agglutination reaction (Table 2). No unrelated peptide competed and the synthetic gp41 fragment, residues 572-591, which is missing the essential carboxyterminal epitope region, did not inhibit agglutination. The inhibition of agglutination with free synthetic antigen was useful in confirming the occasional weak positive samples. If addition of synthetic peptide had failed to inhibit agglutination it would have been indicative of a false positive related to the anti-red blood cell antibody.

Synthetic peptide (0.125 mg/ml) was added to the conjugated antibody prior to the addition of whole blood. The agglutination test was performed as described above. Common sequences are underlined.

**TABLE 2**

| Specificity of peptide inhibition of agglutination | | |
|---|---|---|
| | Added Synthetic Peptide (sequence) | Inhibition of Agglutination |
| | none | 0% |
| gp41 (579-601) | RILAVERYLKDOOLLGIWGCSGK | 100% |
| gp41 (572-591) | GIKQLARILAVERYLKADOO | 0% |
| gp120 (193-200) | ASTTTNYT | 0% |
| gp120 (105-117) | HEDIISLWDQSLK | 0% |
| gp120 (101-118) | VEQMHEDIISLWDOSLKP | 0% |
| gp120 (105-129)Y¹²⁹ | HEDIISLWSQSLKPAVKLTPLCVSY | 0% |

### EXAMPLE 3 - Preparation of Chimeric Antibodies (anti-glycophorin/anti -human D-dimer) and use in assay for D-dimer

Monoclonal antibodies RAT 1C3/86 (anti-human red blood cell) and DD-1C3/108 (anti-human D-dimer as described by Rylatt. et al., 1983, Thrombosis Res., 31, 767-778) were digested with pepsin essentially as described by Hackman, et al., 1981. immunology, 15, 429-436, and purified by chromotrography on a TSK-3000 SW column. 2 mg RAT 1C3/86 was digested for 45 minutes with 1% w/w pepsin in a buffer containing 0.1M acetic acid, 70mM sodium chloride pH 3.5. Meanwhile, 2 mg DD-1C3/108 was digested with 1% w/w pepsin for 2 hours in the same puffer. The reactions were terminated by the addition of 1.5M Tris to raise the pH 8. The F(ab)₂ fragments were purified by gel filtration chromatography on TSK-3000 SW column.

Reduction of the F(ab)₂, and subsequent blocking of the Fab fragment, was carried out as described by Brennan, et al., 1985, Science 229, 81-83. A 3 mg/ml F(ab)₂ preparation was treated with 1mM mercaptoethylamine, in the presence of 10mM sodium arsenite, for 16 hours at 25°C. The Fab fragments were stabilized by reaction with 5.5'-dithiobis (2-nitrobenzoic acid) (Ellman's reagent) for 3 hours at 25°C. The Fab fragment was then purified by gel filtration chromatography on a TSK-3000 SW column.

The thiol form of DD-1C3/108 was regenerated by reaction with 10mM mercaptoethylamine for 30 minutes at 25°C. Excess reagent was removed by gel filtration chromatography on a TSK-3000 SW column. A mixture of the thiol DD-1C3/108 and the Ellman's reagent treated RAT 1C3/86 was incubated for 16 hours at 25°C as described by Brennan, et al. Finally, the chimeric antibody was purified by further gel filtration chromatography on a TSK-3000 SW column.

### Preparation of reagent

Two volumes of 0.1 mg/ml chimeric antibody was mixed with one volume of 7.5 mg/ml unrelated monoclonal antibody (Bruce 5).

### Assay procedure

For assay, 10 µl of heparinized whole blood was placed or a glass slide. 30 µl of reagent was added and mixed. The slide was rocked for three minutes and in the presence of D-dimer agglutination was observed.

### EXAMPLE 4 - Preparation of SPDP-conjugated Digoxin/Anti-Glycophorin antibody conjugate

### Preparation of Digoxin/Ab conjugate

1) Preparation of periodate oxidized digoxin.
   2 ml 100mM sodium periodate was added slowly, dropwise, to 40 mg of digoxin (Sigma), suspended in 2 ml 95% athanol and the reaction was allowed to continue for 30 man at 31°C. The reaction was stooped by the addition of 60 µl of M ethandiol. Finally, the Schiff's base intermediate was stabilized by the addition of 2 ml 40mM cystine (30 min: 37°C) and subsequent reaction With 1 ml of 15 mg/ml sodium borohydride (16h: 25°C).
2) Reduction of cystine/digoxin conjugate.
   3 ml of cystine/digoxin conjugate was reduced by the addition of 40 µl mercaptoethanol (40 min: 37°C) and the product purified by chromatography on a Waters Sep-Pak® C 18 cartridge as described for the reduction of peptide in Example 1 . After rotary evaporation, the sample was reacted with SPDP labelled RAT 1C3/86, which had been labelled with 5 propyldithiopyridine groups/antibody as described in Example 1 (16h: 25°C). Finally, the digoxin/antioody conjugate was purified by gel filtration chromatography on Superose 12.

### EXAMPLE 5 - Preparation of HIV peptide/anti-glycophorin F(ab)₂ conjugate

An alternative reagent for the detection of anti-HIV antibodies uses an F(ab)₂ derivative of the anti-glycophorin antibody.

RAT 1C3/86 (2 mg/ml in 70mM acetate 100mM sodium chloride pH 3.5) was digested with 10 µg/mi pepsin (Sigma P6887) for 40 min at 37°C and the reaction terminated by the addition of 1/10 vol 1.5M Tris base. After overnight dialysis into a buffer containing 5mM sodium phosphate pH 8.0, the antibody fragment was purified by ion-exchange chromatography on DEAE cellulose on a 5-300mM gradient of sodium phosphate pH 8.0.

SPDP labelling of the F(ab)₂ fragment, reduction of the peptide 3.2, conjugation of peptide 3.2 to F(ab)₂ RAT 1C3/86, purification of the peptide conjugate, preparation of reagent for assay and testing procedure were carried out as described for the whole antibody conjugate.

F(ab)₂ conjugates of other erythrocyte or analyte-binding antibodies may similarly be prepared and may be coupled to molecules other than the HIV peptide.

### EXAMPLE 6 - Preparation of HIV pectide/anti-glycophorin Fab' conjugate

Another alternative reagent employs a univalent Fab' fragment as the EBM.

F(ab)₂ RAT 1C3/86 in phosphate buffered saline was incubated with 10mM mercaptoethanol for 1h at room temperature. Then 15mM iodoacetamide was added and the reaction allowed to proceed for 15 min in the dark. Finally, the reaction was terminated by the by dialysis into 100 vol of phosphate buffered saline.

SPDP labelling of the s-carboxymethylated Fab' , reduction of the peptide 3.2, conjugation of peptide 3.2 to the Fab'-TNB (thionitrobenzoyl) RAT fragment of RAT 1C3/86, purification of the peptide conjugate, preparation of reagent for assay and testing procedure were carried out as described for the whole antibody conjugate.

### EXAMPLE 7 - Preparation of Melittin as alternative EBM

A peptide from the bee venom, melittin (CVLTTGLPALISWIKRKRQQ), was used as an alternative to the erythrocyte binding monoclonal antibody. This peptide binds to the erythrocyte surface without lysing the cell (deGrado WF, Kezdy FJ, Kaiser ET. J Am Chem Soc 1981; 103; 679-81). The peptide was synthesised by the Merrifield procedure (Hodges, Merrifield. Anal Biochem 1975; 65; 241).

One advantage of using melittin as the EBM is that it and a peptide-type ABM may be synthesized as a single unit.

### EXAMPLE 8 - Use of Avidin-Biotin linkage to couple EBM and ABM

The ABM and EBM need not be covalently coupled. One alternative is avidin-biotin linkage.

### Preparation of Biotin-labelled melittin

The melittin peptide (10 mg) was reduced with mercaptoethanol as described for the 3.2 peptide in Example 1 . After rotary evaporation, the sample was resuspended in 2 ml 0.1M Tris-HCl , 5mM EDTA pH 8.0 and reacted with 1 ml dimethylsulfoxide (DMSO), containing 4.3 mg N-iodoacetyl-N-biotinylhexylenediamine (Pierce). This was allowed to react for 15 minutes at room temperature and the biotinylated derivative separated from byproducts on a Sephadex® G10 column.

### Preparation of Avidin-labelled peptide 3.2

The 3.2 peptide is coupled to avidin in the same manner as it was coupled to antibody in Example 1.

### Assay

Sub-agglutinating doses of the avidin-labelled peptide are added to the red cells.

### Notes

It will be understood that the avidinated and biotinylated molecules may be interchanged.

## Claims

1. An agglutination reagent for direct detection of an analyte in a blood sample, comprising an erythrocyte binding molecule coupled to an analyte binding molecule, wherein the erythrocyte binding molecule is coupled to the analyte binding molecule in such a manner that the binding characteristics of the binding molecules are not altered by the coupling, and the erythrocyte binding molecule is capable of binding erythrocytes endogenous to the blood sample but does not agglutinate endogenous erythrocytes in the absence of analyte.

2. The reagent of claim 1, wherein said erythrocyte binding molecule is selected from the group consisting of: a non-univalent anti-erythrocyte antibody; a non-univalent fragment of a non-univalent anti-erythrocyte antibody; a fragment of a non-univalent anti-erythrocyte antibody; an F(ab)₂ fragment of an anti-erythrocyte antibody; an Fab' fragment of an anti-erythrocyte antibody; a peptide having an affinity for the erythrocyte membrane but incapable of lysing erythrocytes; a peptide having an affinity for the erythrocyte membrane but incapable of lysing erythrocytes and not derived from an antibody or lectin; a nonlytic, erythrocyte-binding fragment of protamine which does not by itself agglutinate erythrocytes; mellitin; a specific binding fragment of mellitin; a lectin; a specific binding fragment of a lectin; an anti-glycophorin antibody; a specific binding fragment of an anti-glycophorin antibody; an anti-glycophorin A antibody and a specific binding fragment of an anti-glycophorin A antibody.

3. The reagent of claim 1, wherein the erythrocyte binding molecule comprises an anti-erythrocyte antibody, or F(ab)₂ or Fab' fragment thereof and said antibody or F(ab)₂ or Fab' fragment thereof is raised against glycophorin A, glycophorin B, integral membrane protein 1, membrane attached glycoprotein C4, integral membrane glycoprotein, ankyrin, spectrin, glycophorin, glycolipid, glycosphingolipid, protein 4-1 or F-actin.

4. The reagent of claim 1, wherein said erythrocyte binding molecule comprises a non-univalent anti-erythrocyte antibody produced by cell line G26.4.IC3/86 accorded ATCC Deposit No. HB9893 or an F(ab)₂, or Fab' fragment of said antibody.

5. The reagent of of any one of claims 1 to 3, wherein said erythrocyte binding molecule comprises a univalent fragment derived from an anti-erythrocyte antibody and said analyte binding molecule comprises a univalent fragment derived from an anti-analyte antibody.

6. The reagent of any one of claims 1 to 3, wherein the analyte comprises an antibody to HIV.

7. The reagent of any one of claims 1 to 3, wherein the analyte binding molecule comprises a HIV-1 peptide, or hepatitis virus peptide or digoxin or antibody F(ab)₂ or Fab' fragment thereof raised against human D-dimer, or hepatitis virus or an anti-idiotypic antibody.

8. The reagent of claim 7 wherein the HIV-1 peptide comprises gp 41 peptide.

9. The reagent of any one of claims 1 to 8, wherein the erythrocyte binding molecule is attached to the analyte binding molecule by means of covalent coupling.

10. The reagent of any one of claims 1 to 4, wherein the reagent comprises a plurality of different types of conjugates, wherein the analyte binding molecule of each type of conjugate binds to a different epitope of the analyte.

11. The reagent of claim 1, wherein the erythrocyte binding molecule comprises a peptide having an affinity for the erythrocyte membrane but incapable of lysing erythrocytes and not derived from an antibody or lectin.

12. The reagent of claim 11, wherein the peptide comprises a bee venom-like peptide.

13. The reagent of claim 1, wherein the erythrocyte binding molecule but not the analyte binding molecule binds a surface protein or glycoprotein.

14. The reagent of claim 1, wherein the erythrocyte binding molecule comprises a peptide having an affinity for the erythrocyte membrane and the analyte binding molecule is a peptide or protein, and said erythrocyte binding molecule and analyte binding molecule are conjugated by a simple peptide bond.

15. The reagent of claim 14 in which the erythrocyte binding molecule corresponds to a nonlytic, erythrocyte-binding fragment of mellitin which does not by itself agglutinate erythrocytes.

16. The reagent of claim 1 which comprises a heterobifunctional antibody or a heterobifunctional binding fragment of an antibody, said antibody comprising an erythrocyte binding molecule which binds erythrocytes but not the analyte, and a binding molecule binding the analyte but not erythrocytes, the erythrocyte binding molecule being conjugated to the analyte binding molecule by one or more disulfide bonds and not by heterobifunctional coupling agent, said reagent being prepared by forming a heterobifunctional hybrid of a homobifunctional erythrocyte-binding antibody and a homobifunctional analyte-binding antibody, wherein said homobifunctional erythrocyte-binding antibody does not auto-agglutinate erythrocytes.

17. The reagent of claim 16 in which the erythrocyte binding molecule binds glycophorin.

18. The reagent of claim 17 in which the erythrocyte binding molecule comprising an antibody for glycophorin or a specific binding fragment thereof.

19. The reagent of claim 16 in which the analyte-binding molecule binds human D-dimer.

20. The reagent of claim 16 which comprises a heterobifunctional F(ab)₂.

21. The reagent of claim 1 in which the analyte-binding molecule binds an antigen associated with heartworm.

22. The reagent of claim 1, further comprising a secondary antibody or specific binding fragment thereof which specifically recognizes an overlapping epitope formed by the binding of said analyte binding molecule to said analyte.

23. The reagent of claim 22, wherein said secondary antibody is attached to an erythrocyte binding molecule.

24. The reagent of any one of claims 1 to 23, wherein said erythrocytes are human erythrocytes.

25. An agglutination reagent for indirect detection of an analyte in a blood sample, comprising an erythrocyte binding molecule coupled to an analyte analogue wherein said erythrocyte binding molecule is coupled to said analyte analogue in such a manner that binding characteristics of the erythrocyte binding molecule are not altered by the coupling, and said erythrocyte binding molecule is capable of binding erythrocytes endogenous to the sample but does not agglutinate endogenous erythrocytes in the absence of an analyte binding reagent.

26. The reagent of claim 25, wherein said erythrocyte binding molecule is selected from the group consisting of: a non-univalent anti-erythrocyte antibody; a non-univalent fragment of a non-univalent anti-erythrocyte antibody; a fragment of a non-univalent anti-erythrocyte antibody; an F(ab)₂ fragment of an anti-erythrocyte antibody; an Fab' fragment of an anti-erythrocyte antibody; a peptide having an affinity for the erythrocyte membrane but incapable of lysing erythrocytes; a peptide having an affinity for the erythrocyte membrane but incapable of lysing erythrocytes and not derived from an antibody or lectin; a nonlytic, erythrocyte-binding fragment of protamine which does not by itself agglutinate erythrocytes; mellitin; a specific binding fragment of mellitin; a lectin; a specific binding fragment of a lectin; an anti-glycophorin antibody; a specific binding fragment of an anti-glycophorin antibody; an anti-glycophorin A antibody and a specific binding fragment of an anti-glycophorin A antibody.

27. The reagent of claim 26, wherein the erythrocyte binding molecule comprises an anti-erythrocyte antibody, or F(ab)₂ or Fab' fragment thereof and said antibody or F(ab)₂ or Fab' fragment thereof is raised against glycophorin A, glycophorin B, integral membrane protein 1, membrane attached glycoprotein C4, integral membrane glycoprotein, ankyrin, spectrin, glycophorin, glycolipid, glycosphingolipid, protein 4-1 or F-actin.

28. The reagent of claim 25, wherein said erythrocyte binding molecule comprises a non-univalent anti-erythrocyte antibody produced by cell line G26.4.IC3/86 accorded ATCC Deposit No. HB9893 or an F(ab)₂, or Fab' fragment of said antibody.

29. The reagent of any one of claims 25 to 28, wherein the analyte comprises an antibody to HIV.

30. The reagent of claim 26, wherein the erythrocyte binding molecule comprises a peptide having an affinity for the erythrocyte membrane but incapable of lysing erythrocytes and not derived from an antibody or lectin.

31. The reagent of claim 30, wherein the peptide comprises a bee venom-like peptide.

32. The reagent of any one of claims 25 to 31, wherein the erythrocyte binding molecule is attached to the analyte analog by means of covalent coupling.

33. The reagent of claim 26, wherein the erythrocyte binding molecule comprises a peptide having an affinity for the erythrocyte membrane, the analyte analogue is a peptide or protein, and said erythrocyte binding molecule and analyte analogue are conjugated by a simple peptide bond.

34. The reagent of claim 30 or claim 33 in which the erythrocyte binding molecule corresponds to a nonlytic, erythrocyte-binding fragment of mellitin which does not by itself agglutinate erythrocytes.

35. The reagent of any one of claims 25 to 34, wherein said erythrocytes are human erythrocytes.

36. A direct agglutination assay for the presence of an analyte in a blood sample containing erythrocytes which assay comprises mixing the sample with an agglutination reagent according to any one of claims 1 to 24 for a time sufficient for binding to occur to analyte and erythrocytes to cause agglutination; observing whether the erythrocytes are agglutinated and correlating the agglutination with the amount of analyte present in the sample.

37. An indirect agglutination assay for the presence or amount of an analyte in a blood sample which comprises incubating the sample with an agglutination reagent according to any one of claims 25 to 35 and a soluble analyte binding reagent, whereby said agglutination reagent competes with sample analyte for the analyte binding sites of said analyte binding reagent, wherein the incubation is for a time sufficient for analyte to prevent binding of said analyte binding reagent to said analyte analogue to prevent agglutination, observing whether agglutination occurs, and determining the presence or amount of said analyte from the inverse of the degree of agglutination.

38. The assay of claim 36 or 37 in which the sample and the conjugate are contacted essentially only with erythrocytes endogenous to the sample.

39. An agglutination assay test kit comprising an agglutination reagent according to any one of claims 1 to 35 and a reference solution containing a known quantity of analyte.

## Patentansprüche

1. Agglutinationsreagenz zum direkten Nachweis eines Substrats in einer Blutprobe umfaßend: ein EbM, das an ein SbM gebunden ist, wobei das EbM an das SbM so gebunden ist, daß die Bindeeigenschaften der bindenden Moleküle durch die Verbindung nicht verändert werden, und das EbM in der Lage ist, endogene Erythrozyten der Blutprobe zu binden, jedoch nicht zu agglutinieren, wenn kein Substrat vorhanden ist.

2. Reagenz nach Anspruch 1, bei welchem das EbM aus folgender Gruppe ausgewählt ist: ein nicht-einwertiger Anti-Erythrozyten-Antikörper; ein nicht-einwertiges Fragment eines nicht-einwertigen Anti-Erythrozyten-Antikörpers; ein Fragment eines nicht-einwertigen Anti-Erythrozyten-Antikörpers; ein F(ab)₂-Fragment eines Anti-Erythrozyten-Antikörpers; ein Fab'-Fragment eines Anti-Erythrozyten-Antikörpers; ein Peptid, das eine Affinität zur Erythrozytenmembran hat, diese aber nicht lysieren kann; ein Peptid, das eine Affinität zur Erythrozytenmembran hat, diese aber nicht lysieren kann und nicht von einem Antkörper oder Lektin abstammt; ein nicht lysierendes Erythrozyten bindendes Fragment eines Protamin, das nicht allein Erythrozyten agglutiniert; Mellitin; ein spezifisch bindendes Mellitinfragment; ein Lektin; ein spezifisch bindendes Lektinfragment; ein Anti-Glycophorin-Antikörper; ein spezifisch bindendes Fragment eines Anti-Glycophorin-Antikörpers; ein Anti-Glycophorin-A-Antikörper und ein spezifisch bindendes Fragment eines Anti-Glycophorin-A-Antikörpers.

3. Reagenz nach Anspruch 1, bei welchem das EbM aus einem Anti-Erythrozyten-Antikörper oder einem F(ab)₂- oder Fab'-Fragment eines solchen besteht und dieser Antikörper, sein F(ab)₂- oder das Fab'-Fragment speziell gegen Glycophorin A, Glycophorin B, integrales Membranprotein 1, membranständiges Glycoprotein C4, membrandurchspannendes Glycoprotein, Ankyrin, Spektrin, Glycophorin, Glycolipid, Glycosphingolipid, Protein 4-1 oder F-Aktin gezogen wird.

4. Reagenz nach Anspruch 1, bei welchem das EbM einen nicht-einwertigen Anti-Erythrozyten-Antikörper oder die entsprechenden F(ab)2-oder Fab'-Fragmente umfaßt, die von der unter Deposit No. HB9893 bei der ATCC geführten Zellinie G 26.4.IC3/86 gebildet sind.

5. Reagenz nach einem der Ansprüche 1 bis 3, bei welchem das EbM aus einem einwertigen, von einem Anti-Erythrozyten-Antikörper abstammenden Fragment besteht, und das SbM aus einem einwertigen, von einem Anti-Substrat-Antikörper abstammenden Fragment besteht.

6. Reagenz nach einem der Ansprüche 1 bis 3, bei welchem das Reagenz einen Antikörper gegen HIV enthält.

7. Reagenz nach einem der Ansprüche 1 bis 3, bei welchem das SbM aus einem HIV1 Peptid, einem Hepatitisvirus Peptid, einem Digoxin, einem F(ab)₂- Antikörper oder Fab'-Fragment besteht, welches gegen menschliche D-Dimere, Hepatitisviren oder einen Anti-idiotypischen Antikörper gezogen ist.

8. Reagenz nach Anspruch 7, bei welchem das HIV1 Peptid das gp41 Peptid beinhaltet.

9. Reagenz nach einem der Ansprüche 1 bis 8, bei welchem das EbM an das SbM über kovalente Bindungen geheftet ist.

10. Reagenz nach einem der Ansprüche 1 bis 4, bei welchem das Reagenz eine Vielzahl von verschiedenen Konjugattypen enthält, wobei das SbM jedes Konjugattypus an ein anderes Substratepitop bindet.

11. Reagenz nach Anspruch 1, bei welchem das EbM ein Peptid umfaßt, das eine Affinität zur Erythrozytenmembran hat, aber unfähig ist, Erythrozyten zu lysieren und gleichzeitig nicht von einem Antikörper oder Lektin abstammt.

12. Reagenz nach Anspruch 11, bei welchem das Peptid ein bienengiftähnliches Peptid ist.

13. Reagenz nach Anspruch 11, bei welchem das EbM aber nicht das SbM an ein Oberflächenprotein oder Glycoprotein bindet.

14. Reagenz nach Anspruch 1, bei welchem das EbM ein Peptid beinhaltet, das eine Affinität zur Erythrozytenmembran hat, und das SbM ein Peptid oder Protein ist, wobei das EbM und das SbM über eine einfache Peptidbindung verbunden sind.

15. Reagenz nach Anspruch 14, bei welchem das EbM einem nicht lytische Erythrozyten bindenden Fragment des Mellitin entspricht, das nicht selbst Erythrozyten agglutiniert.

16. Reagenz nach Anspruch 1, welches ein heterobifunktionaler Antikörper oder ein heterobifunktional bindendes Fragment eines Antikörpers umfaßt, wobei besagter Antikörper ein EbM, das Erythrozyten aber kein Substrat bindet, und ein Molekül, das Substrat aber keine Erythrozyten bindet, enthält, wobei das EbM über eine oder mehrere Disulfidbrücken an das SbM gekoppelt und nicht über ein heterobifunktionales Bindungsreagenz gebunden ist, und das besagte Reagenz durch Bindung eines heterobifunktionalen Hybrids aus einem homobifunktionalen Erythrozyten-bindenden Antikörper und einem homobifunktionalen Substrat-bindenden Antikörper hergestellt ist, wobei besagter homobifunktionaler Erythrozyten-bindender Antikörper nicht selbstständig mit Erythrozyten agglutiniert.

17. Reagenz nach Anspruch 16, bei welchem das EbM Glycophorin bindet.

18. Reagenz nach Anspruch 17, bei welchem das EbM Glycophorin Antikörper oder ein spezifisch bindendes Fragment eines solchen umfaßt.

19. Reagenz nach Anspruch 16, bei welchem das SbM menschliche D-Dimere bindet.

20. Reagenz nach Anspruch 16, welches heterobifunktionale F(ab)2 umfaßt.

21. Reagenz nach Anspruch 1, in welchem das SbM ein Antigen bindet, das vom Fadenwurm Dirofilaria immitis abgeleitet ist.

22. Reagenz nach Anspruch 1, bei welchem das Reagenz weiterhin einen zweiten Antikörper oder ein spezifisch bindendes Fragment desselben spezifisch erkennt, der ein überlappendes Epitop durch die Bindung von besagten SbM und besagtem Substrat bildet.

23. Reagenz nach Anspruch 22, bei welchem der sekundäre Antikörper an ein EbM gebunden ist.

24. Reagenz nach einem der Ansprüche 1 bis 23, bei welchem die Erythrozyten menschliche Erythrozyten sind.

25. Agglutinationsreagenz für indirekten Nachweis eines Substrats in einer Blutprobe, welches EbM verbunden mit SbM umfaßt, wobei besagtes EbM an besagtes SbM auf eine Art und Weise gebunden ist, daß Bindeeigenschaften des EbM durch die Bindung nicht verändert werden, und wobei besagtes EbM in der Lage ist endogene Erythrozyten der Probe zu binden aber endogene Erythrozyten in Abwesenheit von einem Substrat-bindenden Reagenz nicht agglutiniert.

26. Reagenz nach Anspruch 25, wobei das EbM aus folgender Gruppe ausgewählt ist: ein nicht-einwertiger Anti-Erythrozyten-Antikörper; ein nicht-einwertiges Fragment eines nicht-einwertigen Anti-Erythrozyten-Antikörpers; ein Fragment eines nicht einwertiger Anti-Erythrozyten-Antikörpers; ein F(ab)₂-Fragment eines Anti-Erythrozyten-Antikörpers; ein Fab'-Fragment eines Anti-Erythrozyten-Antikörpers; ein Peptid, das eine hohe Affinität zur Erythrozytenmembran hat, Erythrozyten aber nicht lysieren kann; ein Peptid, das eine hohe Affinität zur Erythrozytenmembran hat, diese aber nicht lysieren kann; ein Peptid, das eine hohe Affinität zur Erythrozytenmembran hat, diese aber nicht lysieren kann und nicht von einem Antikörper oder einem Lektin abstammt; ein nicht-lysierendes Erythrozyten-bindendes Fragment eines Protamin, das allein Erythrozyten nicht agglutiniert; Mellitin; ein spezifisch bindendes Mellitinfragment; ein Lektin; ein spezifisch bindendes Lektinfragment; ein Anti-Glycophorin-Antikörper und ein spezifisch bindendes Fragment eines Anti-Glycophorin-Antikörpers; ein Anti-Glycophorin-A-Antikörper und ein spezifisch bindendes Fragment eines Anti-Glycophorin-A-Antikörpers.

27. Reagenz nach Anspruch 26, bei welchem das EbM aus einem Anti-Erythrozyten-Antikörper oder einem F(ab)₂- oder Fab'-Fragments eines solchen besteht und das F(ab)₂- oder Fab'-Fragment dieses Antikörpers speziell gegen Glycophorin A, Glycophorin B, integrales Membranprotein 1, membranständiges Glycoprotein C4, membrandurchspannendes Glycoprotein, Ankyrin, Spektrin, Glycophorin, Glycolipid, Glycosphingolipid, Protein 4-1 oder F-Aktin gezogen ist.

28. Reagenz nach Anspruch 25, bei welchem das EbM einen nicht einwertigen Anti-Erythrozyten-Antikörper oder das entsprechende F(ab)₂- oder Fab'-Fragment umfaßt, der von der unter Deposit No. HB9893 bei der ATCC geführten Zellinie G 26.4.IC3/86 gebildet wird.

29. Reagenz nach den Ansprüchen 25 bis 28, bei welchem das Substrat einen HIV Antikörper beinhaltet.

30. Reagenz nach Anspruch 26, bei welchem das EbM ein Peptid umfaßt, das eine Affinität zu Erythrozytenmembranen hat, aber nicht in der Lage ist, Erythrozyten zu lysieren, und nicht von einem Antkörper oder Lektin abstammt.

31. Reagenz nach Anspruch 30, welches ein bienengiftähnliches Peptid umfaßt.

32. Reagenz nach den Ansprüchen 25 bis 31, bei welchem das EbM kovalent an ein Substratanalogon gebunden ist.

33. Reagenz nach Anspruch 26, bei welchem das EbM ein Peptid beinhaltet, das eine Affinität zur Erythrozytenmembran hat, und das SbM ein Peptid oder Protein ist, wobei das EbM und das SbM über eine einfache Peptidbindung verbunden sind.

34. Reagenz nach Anspruch 30 oder 33, bei welchem das EbM einem nicht lytischen Erythrozyten-bindenden Fragment des Mellitin entspricht, das nicht selbst agglutiniert.

35. Reagenz nach den Ansprüchen 25 bis 34, bei welchem es sich bei besagten Erythrozyten um menschliche Erythrozyten handelt.

36. Ein direkter Agglutinationstest für die Anwesenheit eines Substrates in Blutproben mit Erythrozyten, welcher Test das Vermischen der Probe mit einem Agglutinationsreagenz gemäß einem der Ansprüche 1 bis 24 für eine Zeitspanne umfaßt, die ausreichend ist, damit zwischen Substrat und Erythrozyten eine Bindung einsetzen kann und so Agglutination verursacht wird; ferner der Test die Beobachtung, ob Erythrozyten agglutiniert sind, sowie das Korrelieren der Agglutination mit der in der Probe vorhandenen Substratmenge umfaßt.

37. Ein indirekter Agglutinationstest für die Anwesenheit eines Substrates in Blutproben, welcher Test das Inkubieren der Probe mit einem Agglutinationsreagenz gemäß der Ansprüche 25 bis 35 und einem Substrat-bindenden Reagenz umfaßt, wobei besagtes Agglutinationsreagenz mit dem Substrat aus der Probe um die Substratbindestellen des besagten substratbindenden Reagenz konkurriert; weiterhin umfaßt der Test eine ausreichend lange Inkubationszeit, damit das Substrat die Bindung des Substratanalogons an das Substrat-bindende Reagenz verhindern kann und damit die Agglutination verhindert; und ferner umfaßt der Test das Beobachten, ob eine Agglutination auftritt, sowie das Bestimmen der Anwesenheit und der Menge des besagten Substrates aus dem umgekehrten Agglutinationsgrad.

38. Test nach Anspruch 36 oder 37, bei dem die Probe und das Konjugat ausschließlich mit endogenen Erythrozyten der Probe in Kontakt kommen.

39. Agglutinationstestkit, welcher ein Agglutinationsreagenz gemäß einem der Ansprüche 1 bis 35 und eine Referenzlösung umfaßt, die eine definierte Menge an Substrat enthält.

## Revendications

1. Un réactif d'agglutination pour la détection directe d'un analyte dans un échantillon sanguin, comprenant une molécule se liant aux érythrocytes couplée à une molécule se liant à l'analyte, dans lequel la molécule se liant aux érythrocytes est couplée à la molécule se liant à l'analyte d'une manière telle que les caractéristiques se liant aux molécules de liaison ne sont pas altérées par le couplage, et que la molécule se liant aux érythrocytes est capable de se lier aux érythrocytes endogènes à l'échantillon sanguin mais n'agglutine pas les érythrocytes endogènes en l'absence d'analyte.

2. Le réactif de la revendication 1, dans lequel ladite molécule se liant aux érythrocytes est choisie dans le groupe constitué : d'un anticorps anti-érythrocyte non monovalent ; d'un fragment non-monovalent d'un anticorps anti-érythrocyte non monovalent ; d'un fragment d'un anticorps anti-érythrocyte non monovalent ; d'un fragment F(ab)₂ d'un anticorps anti-érythrocyte ; d'un fragment Fab' d'un anticorps anti-érythrocyte ; d'un peptide ayant une affinité pour la membrane des érythrocytes mais incapable de lyser les érythrocytes ; d'un peptide ayant une affinité pour la membrane des érythrocytes mais incapable de lyser les érythrocytes et ne provenant pas d'un anticorps ou d'une lectine ; d'un fragment non-lityque se liant aux érythrocytes de protamine qui n'agglutine pas par lui-même les érythrocytes ; de la mellitine ; d'un fragment spécifique se liant à la mellitine ; d'une lectine ; d'un fragment spécifique se liant à une lectine ; d'un anticorps anti-glycophorine ; d'un fragment spécifique se liant à un anticorps anti-glycophorine ; d'un anticorps anti-glycophorine A et d'un fragment spécifique se liant à un anticorps anti-glycophorine A.

3. Le réactif de la revendication 1, dans lequel la molécule se liant aux érythrocytes comprend un anticorps anti-érythrocytes ou un fragment F(ab)₂ ou Fab' de celui-ci, et ledit anticorps ou un fragment F(ab)₂ ou Fab' de celui-ci est produit contre la glycophorine A la glycophorine B, la protéine 1 de membrane intégrale, la glycoprotéine C4 fixée à la membrane, la glycoprotéine intégrale de membrane, l'ankyrine, la spectrine, la glycophorine, un glycolipide, un glycosphingolipide, la protéine 4-1 ou la F-actine.

4. Le réactif de la revendication 1, dans lequel ladite molécule se liant aux érythrocytes comprend un anticorps anti-érythrocytes non monovalent produit par la souche de cellule G26.4.IC3/86 conforme au dépôt ATCC N° HB9893 ou un fragment F(ab)₂ ou Fab' dudit anticorps.

5. Le réactif de l'une quelconque des revendications 1 à 3, dans lequel ladite molécule se liant aux érythrocytes comprend un fragment monovalent dérivé d'un anticorps anti-érythrocytes et ladite molécule se liant à l'analyte comprend un fragment monovalent provenant d'un anticorps anti-analyte.

6. Le réactif de l'une quelconque des revendications 1 à 3, dans lequel l'analyte comprend un anticorps contre HIV.

7. Le réactif de l'une quelconque des revendications 1 à 3, dans lequel la molécule se liant à l'analyte comprend un peptide de HIV-1, ou un peptide de virus d'hépatite, ou de la digoxine, ou un anticorps, ou un fragment Fab' ou F(ab)₂ de celui-ci produit contre le D-dimère humain, ou le virus de l'hépatite ou un anticorps anti-idiotypique.

8. Le réactif de la revendication 7, dans lequel le peptide HIV-1 comprend le peptide gp 41.

9. Le réactif de l'une quelconque des revendications 1 à 8, dans lequel la molécule se liant aux érythrocytes est fixée à la molécule se liant à l'analyte au moyen d'un couplage covalent.

10. Le réactif de l'une quelconque des revendications 1 à 4, dans lequel le réactif comprend un ensemble de types différents de conjugués, dans lequel la molécule se liant à l'analyte de chaque type de conjugué se lie à un épitope différent de l'analyte.

11. Le réactif de la revendication 1, dans lequel la molécule se liant aux érythrocytes comprend un peptide ayant une affinité pour la membrane des érythrocytes mais incapable de lyser les érythrocytes et ne provenant pas d'un anticorps ou d'une lectine.

12. Le réactif de la revendication 11, dans lequel le peptide comprend un peptide analogue au venin d'abeille.

13. Le réactif de la revendication 1, dans lequel la molécule se liant aux érythrocytes mais non la molécule se liant à l'analyte se lie à une protéine ou glycoprotéine de surface.

14. Le réactif de la revendication 1, dans lequel la molécule se liant aux érythrocytes comprend un peptide ayant une affinité pour la membrane des érythrocytes et la molécule se liant à l'analyte est un peptide ou une protéine, et ladite molécule se liant aux érythrocytes et la molécule se liant à l'analyte sont conjuguées à l'aide d'une liaison peptidique simple.

15. Le réactif de la revendication 14, dans lequel la molécule se liant aux érythrocytes correspond à un fragment non-lytiques se liant aux érythrocytes de mellitine qui par elle-même n'agglutine pas les érythrocytes.

16. Le réactif de la revendication 1, qui comprend un anticorps hétérobifonctionnel ou un fragment hétérobifonctionnel se liant à un anticorps, ledit anticorps comprenant une molécule se liant aux érythrocytes qui se lie aux érythrocytes mais pas à l'analyte, et une molécule se liant à l'analyte mais pas aux érythrocytes, la molécule se liant aux érythrocytes étant conjuguée à la molécule se liant à l'analyte par une ou plusieurs liaisons disulfures et non par un agent de couplage hétérobifonctionnel, ledit réactif étant préparé en formant un hybride hétérobifonctionnel d'un anticorps homobifonctionnel se liant aux érythrocytes et d'un anticorps homobifonctionnel se liant à l'analyte, dans lequel ledit anticorps homobifonctionnel se liant aux érythrocytes n'autoagglutine pas les érythrocytes.

17. Le réactif de la revendication 16, dans lequel la molécule se liant aux érythrocytes se lie à la glycophorine.

18. Le réactif de la revendication 17, dans lequel la molécule se liant aux érythrocytes comprend un anticorps contre la glycophorine ou un fragment spécifique se liant à celui-ci.

19. Le réactif de la revendication 16, dans lequel la molécule se liant à l'analyte se lie au D-dimère humain.

20. Le réactif de la revendication 16, qui comprend un F(ab)₂ hétérobifonctionnel.

21. Le réactif de la revendication 1, dans lequel la molécule se liant à l'analyte se lie à un antigène associé avec Dirofilaria immitis.

22. Le réactif de la revendication 1, comprenant en outre un anticorps secondaire ou un fragment spécifique se liant à celui-ci qui reconnait spécifiquement un épitope chevauchant formé par la liaison de ladite molécule se liant à l'analyte auxdits analytes.

23. Le réactif de la revendication 22, dans lequel ledit anticorps secondaire est fixé à une molécule se liant aux érythrocytes.

24. Le réactif de l'une quelconque des revendications de 1 à 23, dans lequel lesdits érythrocytes sont des érythrocytes humains.

25. Un réactif d'agglutination pour une détection indirecte d'un analyte dans un échantillon sanguin, comprenant une molécule se liant aux érythrocytes couplée à un analogue d'analyte dans lequel ladite molécule se liant aux érythrocytes est couplée audit analogue d'analyte d'une manière telle que les caractéristiques se liant à la molécule se liant aux érythrocytes ne sont pas altérées par le couplage, et ladite molécule se liant aux érythrocytes est capable de se lier aux érythrocytes endogènes à l'échantillon mais n'agglutine pas les érythrocytes endogènes en l'absence d'un réactif se liant à l'analyte.

26. Le réactif de la revendication 25, dans lequel ladite molécule se liant aux érythrocytes est choisie dans le groupe constitué : d'un anticorps anti-érythrocyte non monovalent ; d'un fragment non monovalent d'un anticorps anti-érythrocyte non monovalent ; d'un fragment d'un anticorps anti-érythrocyte non monovalent ; d'un fragment F(ab)₂ d'un anticorps anti-érythrocyte ; d'un fragment Fab' d'un anticorps anti-érythrocyte ; d'un peptide ayant une affinité pour la membrane des érythrocytes mais incapable de lyser les érythrocytes ; d'un peptide ayant une affinité pour la membrane des érythrocytes mais incapable de lyser les érythrocytes et ne provenant pas d'un anticorps ou de lectine ; d'un fragment non-lytique se liant aux érythrocytes de protamine qui n'agglutine pas par lui-même les érythrocytes ; de mellitine ; d'un fragment spécifique se liant à la mellitine ; d'une lectine ; d'un fragment spécifique se liant à une lectine ; d'un anticorps anti-glycophorine ; d'un fragment spécifique se liant à un anticorps anti-glycophorine ; d'un anticorps anti-glycophorine A et d'un fragment spécifique se liant à un anticorps anti-glycophorine A.

27. Le réactif de la revendication 26, dans lequel la molécule se liant aux érythrocytes comprend un anticorps anti-érythrocyte, ou un fragment F(ab)₂ ou Fab' de celui-ci et ledit anticorps ou fragment F(ab)₂ ou Fab' de celui-ci est produit contre la glycophorine A, la glycophorine a, la protéine 1 intégrale de membrane, la glycophorine C4 fixée à la membrane, une glycoprotéine intégrale de membrane, l'ankyrine, la spectrine, une glycophorine, un glycolipide, un glycosphingolipide, la protéine 4-1 ou la F-actine.

28. Le réactif de la revendication 25, dans lequel ladite molécule se liant aux érythrocytes comprend un anticorps anti-érythrocyte non monovalent produit par une souche cellulaire G26.4.IC3/86 selon le dépôt ATCC N° HB9893 ou un fragment F(ab)₂ ou Fab' dudit anticorps.

29. Le réactif de l'une quelconque des revendications 25 à 28, dans lequel l'analyte comprend un anticorps contre HIV.

30. Le réactif de la revendication 26, dans lequel la molécule se liant aux érythrocytes comprend un peptide ayant une affinité pour la membrane des érythrocytes mais incapable de lyser les érythrocytes et ne provenant pas d'un anticorps ou d'une lectine.

31. Le réactif de la revendication 30, dans lequel le peptide comprend un peptide analogue au venin d'abeille.

32. Le réactif de l'une quelconque des revendications 25 à 31, dans lequel la molécule se liant aux érythrocytes est fixée à l'analogue d'analyte au moyen d'un couplage covalent.

33. Le réactif de la revendication 26, dans lequel la molécule se liant aux érythrocytes comprend un peptide ayant une affinité pour la membrane des érythrocytes, l'analogue d'analyte est un peptide ou une protéine, et ladite molécule se liant aux érythrocytes et l'analogue d'analyte sont conjugués à l'aide d'une liaison peptidique simple.

34. Le réactif de la revendication 30 ou de la revendication 33, dans lequel la molécule se liant aux érythrocytes correspond à un fragment non-lityque se liant aux érythrocytes de mellitine qui n'agglutine pas par elle-même les érythrocytes.

35. Le réactif de l'une quelconque des revendications 25 à 34, dans lequel lesdits érythrocytes sont des érythrocytes humains.

36. Un dosage direct par agglutination pour la présence d'un analyte dans un échantillon sanguin contenant des érythrocytes, dosage qui comprend le mélange de l'échantillon avec un réactif d'agglutination conforme à l'une quelconque des revendications 1 à 24 pendant un temps suffisant pour qu'une liaison apparaisse entre l'analyte et les érythrocytes pour provoquer une agglutination ; l'observation à savoir si les érythrocytes sont agglutinés, et la corrélation de l'agglutination avec la quantité d'analytes présents dans l'échantillon.

37. Un dosage d'agglutination indirect pour la présence ou la quantité d'un analyte dans un échantillon sanguin qui comprend l'incubation de l'échantillon avec un réactif d'agglutination conforme à l'une quelconque des revendications 25 à 35 et un réactif soluble se liant à l'analyte, de sorte que ledit réactif d'agglutination entre en concurrence avec l'analyte de l'échantillon pour les sites se liant à l'analyte dudit réactif se liant à l'analyte, dans lequel l'incubation est réalisée pendant un temps suffisant pour que l'analyte empêche la liaison dudit agent se liant à l'analyte audit analogue d'analyte pour empêcher l'agglutination, l'observation à savoir si une agglutination apparaît et la détermination de la présence ou de la quantité dudit analyte à partir de l'inverse du degré d'agglutination.

38. Le dosage de la revendication 36 ou 37, dans lequel l'échantillon et le conjugué sont essentiellement mis en contact seulement avec des érythrocytes endogènes à l'échantillon.

39. Une trousse d'essai de dosage d'agglutination comprenant un réactif d'agglutination conforme à l'une quelconque des revendications 1 à 35 et une solution de référence contenant une quantité connue d'analyte.
